# EUROPEAN PATENT APPLICATION

(11) **EP 3 546 593 A1**
(43) Date of publication of application: **02.10.2019**
(21) Application number: 17874745.7
(22) Date of filing: 27.11.2017
(51) Int. Cl.: C12Q 1/68

(54) **CHEMOENDOCRINE SCORE (CES) BASED ON PAM50 FOR BREAST CANCER WITH POSITIVE HORMONE RECEPTORS WITH AN INTERMEDIATE RISK OF RECURRENCE**

(30) Priority: 28.11.2016 ES 201601012
(71) Applicant: Geicam (Grupo Español de Investigación en Cancer de Mama), 28700 San Sebastián de los Reyes (ES)
(72) Inventor: PRAT, Aleix, 08036 Barcelona (ES); PEROU, Charles M., Chapel Hill, NC 27599-7299 (US); ALBA CONEJO, Emilio, 29010 Málaga (ES)
(74) Representative: Hoffmann Eitle
(86) International application number: PCT/ES2017/000148
(87) International publication number: WO 2018/096191

(57) **Abstract**

The present invention refers to the development of a Chemo Endocrine Score (CES), based on the well-known PAM50 assay, for predicting whether a breast cancer patient is responsive to a chemotherapy or endocrine treatment, particularly in an HR+/HER2- breast patient beyond PAM50 Risk of Relapse (ROR) and intrinsic subtypes. Particularly, the clinical utility of this CES predictor lies in the PAM50 ROR-intermediate group, where the proportion of each CES group (endocrine-sensitive, intermediate and chemo-sensitive) is more than 25%.

## Description

The invention relates to the field of treatment efficacy prediction in patients with breast cancer. More particularly, this invention relates to the prediction of the efficacy of a chemo- or endocrine-treatment in hormone receptor-positive (HR+, "Hormone Receptors +") and HER2-negative (HR+/HER2-) breast cancer patients beyond intrinsic subtype and Risk of Relapse (ROR), preferably wherein the patient is classified as intermediate risk of relapse.

### STATE OF ART

Approximately 70% of invasive breast cancers at diagnosis are HR+/HER2- (TCGA: Comprehensive molecular portraits of human breast tumours. Nature 2012; 490:61-70; Prat A, et al. Journal of Clinical Oncology. 2013, 31:203-209). However, HR+/HER2- disease is clinically and biologically heterogeneous and further sub classifications are needed to better tailor current and future treatments (Ades F., et al. Journal of Clinical Oncology. 2014, 32:2794-2803; Prat A, et al. Mol Oncol. 2011, 5:5-23, Prat A, et al. Nat Rev Clin Oncol 2012, 9:48-57).

Over the last decade, molecular characterization studies have identified and extensively investigated the two main molecular subtypes within HR+/HER2- disease (i.e. Luminal A and B) (The Cancer Genome Atlas - TCGA: Comprehensive molecular portraits of human breast tumours. Nature. 2012, 490:61-70; Prat A, et al. Journal of Clinical Oncology. 2013. 31:203-209; Perou CM, et al. Nature. 2000, 406:747-752). Luminal A tumors have an improved prognosis at 5- and 10-year follow-up compared with Luminal B tumors irrespective of classical clinical-pathological variables (e.g. tumor size and nodal status) and (neo)adjuvant treatment (i.e. endocrine and chemotherapy) (TCGA: Comprehensive molecular portraits of human breast tumours. Nature 2012; 490:61-70; Prat A, et al. Journal of Clinical Oncology. 2013, 31:203-209, Martin M, et al. Breast Cancer Research and Treatment. 2013, 138:457-466, Prat A, et al. Journal of the National Cancer Institute. 2014, 106). In terms of treatment sensitivity, Luminal A tumors achieve significant lower rates of pathological complete response (pCR) than Luminal B tumors following neoadjuvant multi-agent chemotherapy (Usary J, et al. Clinical Cancer Research. 2013, 19:4889-4899; von Minckwitz G, et al. Journal of Clinical Oncology. 2012, 30:1796-1804; Prat A, et al. Breast Cancer Research and Treatment. 2012, 135:301-306; Prat A, et al. BMC Medicine. 2015, 13:1-11). However, less clear is the difference in endocrine sensitivity between the two luminal subtypes (Ellis MJ, et al. Journal of Clinical Oncology. 2011, 29:2342-2349; Dunbier AK, et al. Steroids. 2011,76:736-740).

Today, adjuvant endocrine therapy for 5-10 years is recommended for all patients with HR+/HER2- early breast cancer, whereas chemotherapy is recommended for patients with intermediate and high risk tumors (Goldhirsch A, et al. Personalizing the treatment of women with early breast cancer: highlights of the St Gallen International Expert Consensus on the Primary Therapy of Early Breast Cancer 2013. Annals of Oncology). However, the relationship between therapy and risk warrants further study considering that risk is associated with both factors related to tumor biology and clinical-pathological features such as tumor size and nodal status, whereas therapy responsiveness is generally considered to be independent of clinical pathological factors.

Accordingly, there persists a need in the art for tools for identifying the heterogeneous subgroups having HR+/HER2- breast cancer with different prognostic and treatment sensitivities in order to select the most suitable type and extent of treatment for the patient.

### DESCRIPTION OF THE INVENTION

Hormone receptor-positive (HR+) breast cancer is clinically and biologically heterogeneous and subgroups with different prognostic and treatment sensitivities need to be identified. Here we present the development and clinical validation across multiple studies of a gene expression-based predictor, based on the well-known PAM50 assay that is associated with chemotherapy and endocrine therapy response in early breast cancer beyond PAM50 Risk of Relapse (ROR) and intrinsic subtypes. The clinical utility of this PAM50-based Chemo-Endocrine Score (CES) predictor lies in the PAM50 ROR-intermediate group, where the proportion of each CES group (endocrine-sensitive, intermediate and chemo-sensitive) is more than 25%.

CES is a single genomic signature capable of measuring chemoendocrine sensitivity in HR+/HER2- breast cancer beyond intrinsic subtype, other genomic signatures, and the standard pathology variables. CES could be of particular clinical value in patients with HR+/HER2-intermediate risk disease where the benefit of adjuvant multiagent chemotherapy is unclear. A CES equal or higher than 0.7 is indicative that said patient is responsive to endocrine treatment (CES-E), and wherein a CES equal or lower than 0.3 is indicative that said patient is responsive to chemotherapy treatment (CES-C). Of note, a CES-U group seems to be a genuine grey area where decisions regarding the need of chemotherapy might be difficult.

Our results are the first to confirm, in a randomized setting, an inverse relationship of endocrine and chemotherapy sensitivity in Estrogen Receptor positive (ER+) breast cancer. Previous evidences suggested an inverse relationship between proliferation- and ER-related biological processes regarding endocrine and chemotherapy sensitivity of ER+ breast cancer. For example, two independent studies showed an inverse correlation between a 200-gene ER reporter score, or between TAU expression, an ER-related gene, and endocrine sensitivity and chemosensitivity (Symmans WF, et al. Journal of Clinical Oncology. 2010, 28:4111-4119; Andre F, et al. Clinical Cancer Research. 2007, 13:2061-2067). In addition, high recurrence score measured by Oncotype DX (Genomic Health, Inc., Redwood, CA) predicted little or no benefit from adjuvant tamoxifen therapy in the NSABP-B14 trial, but at the same time also predicted substantial benefit from adjuvant CMF (Cyclophosphamide Methotrexate Fluorouracil) chemotherapy in the NSABP-B20 trial (Paik S, et al. New England Journal of Medicine. 2004, 351:2817-2826; Paik S, et al. Journal of Clinical Oncology. 2006, 24:3726-3734). These results fit with our results showing that virtually all patients with ROR-high disease are identified as CES-C; however, the present invention also highlights that within ROR-high/CES-C disease not all ER+/HER2- samples are luminal (i.e. Luminal A or B) since non-luminal disease (i.e. Basal-like and HER2-enriched) can also be identified. According to our results, the chemotherapy benefit in ROR-high/non-luminal tumors within HR+/HER2- disease is likely even greater than in ROR-high/Luminal B tumors.

The results of the present invention also suggest that a main driver of endocrine therapy sensitivity and chemotherapy sensitivity within ER+/HER2- disease is the Basal-like *versus* Luminal A intrinsic biology. To capture both biological states in each individual sample, it was calculated the correlation coefficients (CC) of each sample to both PAM50-centroids (i.e. Luminal A and Basal-like) and then subtracted both coefficients. Thus, instead of choosing a gene signature (e.g. a proliferation-based signature) of the many signatures that can discriminate between both subtypes in one way or another, it was decided to incorporate into a score the Basal-like vs. Luminal A intrinsic state of each tumor as identified by the PAM50 subtype predictor. Of note, the PAM50 genes were originally selected for their ability to capture the intrinsic biology displayed by 1,900 genes (i.e. the so-called intrinsic gene list). In fact, in the TCGA, intrinsic subtype defined by PAM50 captured the vast majority of the biological diversity displayed by most molecular data-types analyzed (TCGA: Comprehensive molecular portraits of human breast tumours. Nature. 2012, 490:61-70).

From a clinical perspective, the data of the present invention support current breast cancer guidelines for the systemic treatment of early HR+/HER2- breast cancer. On one hand, patients with a low-ROR score and a low tumor burden (i.e. <10% risk of distant relapse at 10 years) are recommended to be treated with endocrine therapy-only (Harris LN, et al. Journal of Clinical Oncology. 2016). Indeed, the results shown in the present invention suggest that these patients have tumors that are highly endocrine sensitive and have low chemotherapy sensitivity. On the other hand, patients with high-risk HR+/HER2-disease are recommended to be treated with endocrine therapy and chemotherapy. According to the data of the present invention, this group is the one with high chemotherapy benefit and low endocrine benefit. Regarding endocrine therapy in this group, the main issue is that we do not have survival data suggesting that CES-C tumors do not benefit at all from endocrine therapy. Therefore, withdrawal of a potentially efficacious treatment strategy such as endocrine therapy in a patient with an ER+ tumor (as defined by the American Society of Clinical Oncology / College of American Pathologist - ASCO/CAP - guidelines) that is identified as CES-C or ROR-high should not be recommended today, although in patients whose tumors contain low levels of ER (1% to 10%), ASCO/CAP recommend to discuss the pros and cons of endocrine therapy. A large randomized adjuvant trial involving thousands of patients to answer this particular question is unlikely to happen.

Although the clinical implications of CES in low and high risk HR+/HER2- disease are minimal, the observation that intermediate risk HR+/HER2- disease, which represents ∼30% of newly diagnosed breast cancer, is biologically heterogeneous with a range of chemotherapy sensitivities might have implications for the interpretation of two ongoing prospective clinical trials. In the TailorX phase III trial, 4,500 patients with HR+/HER2- node-negative early breast cancer with intermediate RS have been randomized to adjuvant chemotherapy or no chemotherapy. According to our analysis, this intermediate group might be composed of at least 3 groups with different chemotherapy sensitivities. Of note, the CES-U group seems to be a genuine grey area where decisions regarding the need of chemotherapy might be difficult. A similar situation might occur in the RxPONDER phase III clinical trial where patients with HR+/HER2- early breast cancer, and 1-3-positive lymph nodes, with low/intermediate risk are being randomized to adjuvant chemotherapy or not. A potential explanation is that OncotypeDX RS, as well as other prognostic gene expression-based tests, such as PAM50 ROR or MammaPrint43, have been specifically designed or trained to predict outcome and not intrinsic tumor biology or treatment sensitivity. Although a strong negative correlation is observed between ROR (risk) and CES (drug sensitivity), there are substantial differences between them at the individual level (-40% discordance).

There are several caveats to our study. First, this is a retrospective study involving heterogeneous patient populations and the results need to be confirmed in a prospective clinical trial(s). Second, although the data presented here validates CES from a clinical perspective, further analytical validation will be needed since in most datasets, except the Malaga set, the research-based version of PAM50 was used. However, the fact that CES (as a continuous variable and the 2 cut-points) predicted pCR in the Malaga set suggests that analytical validation of this biomarker is feasible. Third, we did not evaluate the association of CES with survival data from a randomized clinical trial of adjuvant chemotherapy vs no adjuvant chemotherapy, or adjuvant endocrine therapy versus no adjuvant endocrine therapy. Thus, the predictive value of these signatures was only evaluated in the neoadjuvant setting where different tumor response endpoints were evaluated, most of which have been associated with patient survival (Ogston KN, et al. The Breast. 2003, 12:320-327; Cortazar P, et al. The Lancet. 2014, 384:164-172). Fourth, some of the signatures evaluated in the MDACC-based dataset, such as OncotypeDX recurrence score or genomic grade index, were derived from microarray-based data and thus are not the commercially available versions. Fifth, we were not able to demonstrate a consistent association of CES with endocrine response in HR+ disease after excluding the HER2-positive cases. In the Edinburgh dataset, HER2 status was not available for all patients. Although we derived an ERBB2 expression based surrogate definition of HER2 status and showed that CES is independently associated with response, this was not prespecified and does not meet REMARK guidelines ("REporting recommendations for tumour MARKer prognostic studies", McShane LM et al. Br J Cancer. 2005 Aug 22; 93(4): 387-391). In addition, the association of CES with endocrine response did not reach statistical significance (p=0.09) in patients with HR+/HER2-negative disease in the Marsden dataset. Finally, patients from each of the datasets received different anthracycline/taxane-based chemotherapy regimens, schedules and doses, and thus the ability of the signatures to predict response to particular chemotherapeutics or treatment regimens could not be tested.

Another important consideration of the present invention is that we did not attempt to identify an optimal cutoff(s) for CES but rather focused on the association of the continuous expression of CES with each endpoint. The main reason is that different gene expression-based platforms and protocols were used in each cohort and thus, standardization of a biomarker cut-point would have been difficult to achieve and most likely unreliable. In any case, the fact that all four testing sets gave very similar associations, and were found independently of the platform/protocol used, argues in favor of a robust finding.

To conclude, CES is a single genomic signature capable of measuring chemoendocrine sensitivity in HR+/HER2- breast cancer beyond intrinsic subtype, other genomic signatures, and the standard pathology variables. CES could be of particular clinical value in patients with HR+/HER2- intermediate risk disease where the benefit of adjuvant multiagent chemotherapy is unclear.

According to the information shown in the present invention, a first aspect of the present invention relates to an *in vitro* method for predicting whether a breast cancer patient is responsive to a chemotherapy or endocrine treatment in an HR+/HER2- isolated sample of the patient classified as ROR intermediate group by the PAM50 kit, wherein said method comprises:
a) obtaining by the PAM50 kit, the correlation coefficient corresponding to the sample classified as Luminal A intrinsic subtype and the correlation coefficient corresponding to the sample classified as Basal-Like intrinsic subtype, in the isolated sample, and
b) obtaining the Chemo Endocrine Score (CES) by subtracting the correlation coefficient corresponding to the sample classified as Basal-Like subtype from the correlation coefficient corresponding to the sample classified as Luminal A subtype; (CES=CC Luminal A - CC Basal-Like)
wherein a CES equal or higher than 0.7 is indicative that said patient is responsive to endocrine treatment (CES-E), and wherein a CES equal or lower than 0.3 is indicative that said patient is responsive to chemotherapy treatment (CES-C).

In a preferred embodiment of the first aspect of the present invention, the isolated sample is a biopsy sample.

In a second aspect of the present invention, relates to the *in vitro* use of the CES for predicting whether a breast cancer patient is responsive to a chemotherapy or endocrine treatment in an HR+/HER2- isolated sample of the patient classified as ROR intermediate group by the PAM50 kit.

In a preferred embodiment, the CES is obtained by subtracting the correlation coefficient corresponding to the sample classified as Basal-Like subtype from the correlation coefficient corresponding to the sample classified as Luminal A subtype, by the PAM50 kit.

In another preferred embodiment of the present aspect of the invention, it is noted that wherein a CES equal or higher than 0.7 is indicative that said patient is CES-E. In another preferred embodiment, wherein a CES equal or lower than 0.3 is indicative that said patient is CES-C.

In another preferred embodiment, the isolated sample is a biopsy sample.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

**Fig 1****.** Gene expression association with either chemotherapy or endocrine therapy sensitivity: association between the expression of each individual gene (n=542) and Miller-Payne response in each arm of the GEICAM 2006-03 trial. Selected top genes whose expression is found significantly associated with response are shown on the right.
**Fig 2** Gene expression association with either chemotherapy or endocrine therapy sensitivity: mean expression of the Top 50 genes associated with endocrine sensitivity (upper panel) and chemotherapy sensitivity (bottom panel) in the GEICAM 2006-03 trial across the intrinsic subtypes of breast cancer. The RNAseq-based gene expression data has been obtained from the The Cancer Genome Atlas breast cancer project data portal (https://tcgadata.nci.nih.gov/tcga/).
**Fig 3****.** Gene expression association with either chemotherapy or endocrine therapy sensitivity: significance and scoring of the CES.
**Fig 4****.** CES (as continuous variable) association with response to chemo or endocrine therapy in the 4 validation datasets
**Fig 5****.** Association of CES with Miller-Payne following chemotherapy in HR+/HER2-negative disease from the Malaga-based cohort.
**Fig 6****.** CES association with endocrine sensitivity in the Edinburgh dataset (n=120): tumor volume changes of each patient and response classification.
**Fig 7****.** CES association with endocrine sensitivity in the Edinburgh dataset (n=120): association of CES and other variables with response (defined as at least 70% reduction by 90 days) in the overall population.
**Fig** 8. CES association with endocrine sensitivity in the Edinburgh dataset (n=120): association of CES and other variables with response within HER2-negative disease.

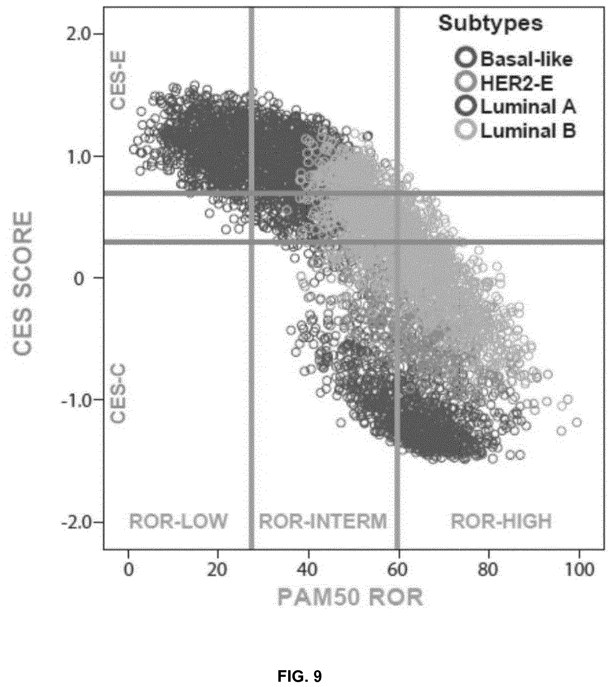

**Fig 9****.** Prognosis (PAM50 ROR), intrinsic subtype and CES in 6,007 primary breast cancers: a scatter plot of CES score and ROR score, colored by subtype, is shown. The two horizontal lines indicate the cutoffs of each CES group. The two vertical lines indicate the cutoffs of each PAM50 ROR group.

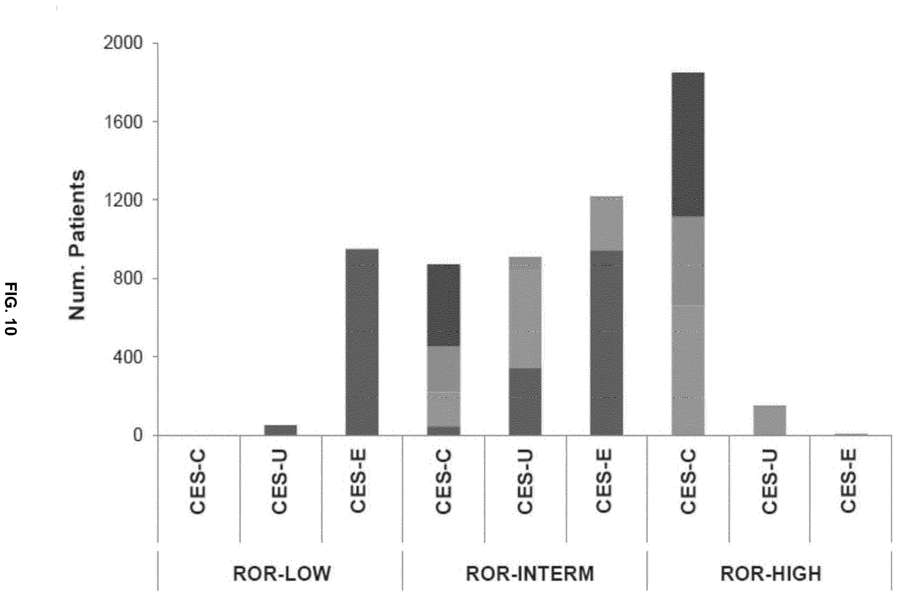

**Fig 10****.** Number of patients in each CES group based on ROR. Each bar is colored by subtype.
**Fig 11****.** Survival outcomes in HR+ early breast cancer with ROR-intermediate: node-negative disease treated without adjuvant systemic therapy.
**Fig 12****.** Survival outcomes in HR+ early breast cancer with ROR-intermediate: node-negative and node-positive disease treated with adjuvant tamoxifen-only.
**Fig 13****.** Survival outcomes in HR+ early breast cancer with ROR-intermediate: node-positive disease treated with adjuvant chemotherapy and endocrine therapy in the GEICAM/9906 clinical trial.
**Fig 14****.** Survival outcomes in HR+ early breast cancer with ROR-intermediate: node-negative and node-positive disease treated with neoadjuvant chemotherapy and adjuvant endocrine therapy.

### EXAMPLES

### Methods and Materials

### GEICAM/2006-03 clinical trial

Pre-treatment core biopsy samples from patients recruited in the luminal cohort of the GEICAM/2006-03 phase II neoadjuvant clinical trial (NCT00432172) were evaluated (Alba E, et al. Annals of Oncology. 2012). In this study, 95 patients with estrogen receptor (ER)-positive (Allred 3-8), progesterone receptor (PR)-positive (Allred 3-8), HER2- (according to the ASCO/CAP guidelines (Wolff AC, et al. Journal of Clinical Oncology 25:118-145, 2006)), and cytokeratin8/18-positive breast cancer were randomly assigned to receive 24 weeks of neoadjuvant chemotherapy or endocrine therapy. Chemotherapy consisted of epirubicin 90 mg/m² intravenously (i.v.) combined with cyclophosphamide 600 mg/m² i.v. on day 1 every 21 days, for 4 cycles followed by docetaxel 100 mg/m² administered i.v. on day 1 every 21 days for 4 cycles. Endocrine therapy consisted of oral exemestane 25 mg daily. Premenopausal patients received goserelin 3.6 mg subcutaneously every 28 days for 6 doses. After neoadjuvant treatment, patients underwent mastectomy or conservation surgery plus axillary lymph node dissection (unless previous negative sentinel lymph node biopsy).

### GEICAM 2006-03 pathological response end-point

The 5-point scale Miller and Payne histological grading system (Ogston KN, et al. The Breast. 2003, 12:320-327) was used to measure tumor response. This grading system is a 5-point scale, which focuses on reduction in tumor cellularity in the treated breast tumor (at surgery) compared to the pre-treatment samples. Grade 1: no change in overall cellularity. Grade 2: up to 30% loss in cellularity. Grade 3: between an estimated 30% and 90% reduction in tumor cells. Grade 4: more than 90% loss of tumor cells. Grade 5: pathological complete response (pCR). Ductal carcinoma in situ may be present. In this study, the Miller and Payne scale was reduced to a 3-point scale in order to have a fair number of cases in each category and arm: no response (grade 1 and 2), intermediate response (Grade 3) and high response (Grade 4 and 5).

### GEICAM 2006-03 gene expression analysis

Sixty-three of 95 pre-treatment tumor samples were available for gene expression analyses. For each sample, a section of the formalin-fixed paraffin-embedded (FFPE) breast tissue was first examined with hematoxylin and eosin staining to confirm the diagnosis and determine the tumor area. Two 1 mm cores enriched with tumor tissue were obtained from the original tumor block and a minimum of -100 ng of total RNA was purified to measure the expression of 543 breast cancer-related genes using the nCounter platform (Nanostring Technologies, Seattle, WA, US). Data was log base 2 transformed and normalized using 5 house-keeping genes (ACTB, MRPL19, PSMC4, RPLP0 and SF3A1) and 14 negative and positive controls using the nCounter platform (Nanostring Technologies, Seattle, WA, US) (Geiss GK, et al. Nat Biotech. 2008, 26:317-325).

### Independent/testing datasets

Gene expression and response data were evaluated from 4 independent neoadjuvant datasets (Dunbier AK, et al. Steroids. 2011, 76:736-740; Hatzis C, et al. Jama. 2011, 305:1873-1881; Prat A, et al. Clinical Cancer Research. 2015; Dunbier AK, et al Journal of Clinical Oncology. 2010, 28:1161-1167; Smith IE, et al. Journal of Clinical Oncology. 2007, 25:3816-3822; Turnbull AK, et al. Journal of Clinical Oncology. 2015). Gene expression and survival data were evaluated from 4 independent datasets of patients with early breast cancer (Prat A, et al. Journal of Clinical Oncology. 2013, 31:203-209; Hatzis C, et al. Jama. 2011, 305:1873-1881; Fan C, et al. BMC Medical Genomics. 2011, 4:1-15; Prat A, et al. Annals of Oncology. 2012, 23:2866-2873).

### Hatzis independent dataset

We evaluated the publicly available microarray gene expression-based dataset (GSE25066) reported by Hatzis et al. (Hatzis C, et al. Jama. 2011, 305:1873-1881) that includes 508 patients (272 HR+/HER2- disease) treated with multi-agent neoadjuvant chemotherapy in various research protocols: LAB99-402, USO-02-103, 2003-0321 and I-SPY-1. The vast majority of patients (96.4%) received sequential anthracycline/taxane-based regimens. Gene expression was performed in the pre-treatment samples and tumor response data after chemotherapy (i.e. pCR in the breast/axilla versus not) was available for 488 patients (260 with HR+/HER2-disease).

### Malaga independent dataset

A total of 216 locally assessed HR+/HER2- banked tumor samples from breast cancer patients in an independent multi-center Spanish cohort were selected as previously reported (Prat A, et al. Clinical Cancer Research, 2015). All patients had been prescribed a standard neoadjuvant chemotherapy regimen consisting of 8-10 cycles of anthracyclines and taxanes. Pre-treatment tumor samples were tested with the standardized PAM50 assay (PROSIGNA®) using the nCounter diagnostic platform. Gene expression in the pre-treatment samples was performed at Malaga University Hospital, data was normalized by Nanostring Technologies and CES was applied at VHIO blinded from clinical data. Tumor response data (i.e. pCR in the breast/axilla vs. not) was available for 180 patients. In addition, Miller-Payne response data was available for 171 patients.

### Marsden independent dataset

We evaluated the RNA profiles (HumanWG-6 v2 Expression BeadChips [Illumina, San Diego, CA]) obtained from pretreatment core-cut tumor biopsies from 103 postmenopausal patients with primary ER+ breast cancer treated with neoadjuvant anastrozole for 16 weeks in a phase II clinical trial (Dunbier AK, et al. Steroids. 2011, 76:736-740; Dunbier AK, et al. Journal of Clinical Oncology. 2010, 28:1161-1167; Smith IE, et al. Journal of Clinical Oncology, 2007, 25:3816-3822). A subgroup of patients received gefitinib during the first 2 weeks; however, the addition of gefitinib to anastrozole had no additional clinical or biological effect on Ki679. Clinical tumor response (complete and partial response versus stable and progressive disease) was used as the endpoint. CES was calculated at IDIBAPS (Institut d'Investigacions Biomèdiques August Pi i Sunyer, Barcelona) blinded from clinical data.

### Edinburgh independent dataset

We evaluated the RNA profiles (HumanWG-6 v2 Expression BeadChips [Illumina, San Diego, CA] and Affymetrix U133A 2.0 [Santa Clara, California, USA]) obtained from pretreatment core-cut tumor biopsies from 120 postmenopausal patients with primary ER+ breast cancer treated with neoadjuvant anastrozole for at least 12 weeks (Turnbull AK, et al. Journal of Clinical Oncology, 2015). Response was evaluated by imaging ultrasound. Clinical tumor response was defined as tumor volume shrinkage of at least 70% by 90 days of treatment. Raw gene expression data can be found in Gene Expression Omnibus (GSE55374 and GSE20181). HER2 clinical status was available for 45 cases. HER2+ cases showed higher expression of ERBB2 compared to HER2-negative cases. We used percentile 80 as a cutoff to define HER2 positivity in those cases without clinical HER2 status. A total of 89 cases were found to be HER2-negative.

### Intrinsic subtype assignment

All tumors were assigned to an intrinsic molecular subtype of breast cancer (Luminal A, Luminal B, HER2-enriched, Basal-like) and the normal-like group using the research- based PAM50 subtype predictor (Parker JS, et al. J Clin Oncol. 2009, 27:1160-1167; Nielsen TO, et al. Clin Cancer Res. 2010, 16:5222-5232, except for the Malaga cohort where the PAM50 standardized and commercial nCounter-based assay was used. Before subtyping, each individual dataset was normalized accordingly as previously reported (Prat A, et al. Br J Cancer. 2014, 111:1532-1541, 2014), except for the Malaga cohort that was normalized by Nanostring according to their algorithm. Of note, the Edinburgh microarray-based dataset is composed of ER+ samples-only and proper centering for intrinsic subtyping calling was not possible (Prat A, et al. Nat Rev Clin Oncol. 2012, 9). In this dataset, CES was evaluated as a continuous variable since it is not affected by centering.

### Adjuvant datasets

Gene expression and survival data were evaluated from 4 independent datasets of patients with early breast cancer with ROR-intermediate disease (Wolff AC, et al. Journal of Clinical Oncology, 2006, 25:118-145; Hatzis C, et al. Jama. 2011, 305:1873-1881; Fan C, et al. BMC Medical Genomics. 2011, 4:1-15; Prat A, et al. Annals of Oncology. 2012, 23:2866-2873). One is the MDACC-based dataset previously described where distant relapse-free survival was recorded (Hatzis C, et al. Jama. 2011, 305:1873-1881, 2011). All patients received neoadjuvant chemotherapy and endocrine therapy. The second one is a combined and previously published cohort of 1,318 patients with HR+ disease treated with adjuvant tamoxifen-only (Prat A, t al. Annals of Oncology. 2012, 23:2866-2873). The third one is a combined and previously published cohort of patients who did not receive any adjuvant systemic therapy (Fan C, et al. BMC Medical Genomics. 2011, 4:3). Finally, we analyzed the samples, as previously described (Prat A, et al. Br J Cancer. 2014, 111:1532-1541), from the GEICAM/9906 clinical trial, where all patients received adjuvant multi-agent chemotherapy and endocrine therapy (Prat A, et al. J Clin Oncol. 2013, 31:203-9).

### Combined cohort of primary breast cancer

To evaluate the relationship between PAM50 subtype calls, prognosis (ROR-P: ROR-Prognosis) and CES, we combined PAM50 data from 7 independent and previously reported cohorts (TCGA: Comprehensive molecular portraits of human breast tumours. Nature, 2012 490:61-70; Prat A, et al. Journal of Clinical Oncology. 2013, 31:203-209; Prat A, et al. Breast Cancer Research and Treatment. 2012, 135:301-306; Hatzis C, et al. Jama. 2011, 305:1873-1881; Curtis C, et al. Nature. 2012, 486:346-352; Horak CE, et al. Clinical Cancer Research. 2013, 19:1587-1595; Fan C, et al. BMC Medical Genomics. 2011, 4:3) representing a total of 6,007 primary tumor samples. CES was evaluated in each individual cohort, and a combined matrix was created.

### Statistical analysis

Biologic analysis of gene lists was performed with DAVID 6.7 annotation tool (Dennis G, et al. Genome Biol. 2003, 4:R60) using the 543-gene list as background. Association between the expression of each gene and Miller-Payne response (3 categories) was assessed by a quantitative Significance Analysis of Microarrays (SAM) (Tusher VG, et al. Proc Natl Acad Sci USA. 2001, 98:5116-5121). In both testing datasets, association between each variable and pCR or clinical/radiological response was assessed by univariate and multivariable logistic regression analyses. The predictive performance of CES was evaluated using receiver operating characteristic (ROC) curve analysis. Estimates of survival were from the Kaplan-Meier curves and tests of differences by the log-rank test. Univariate and multivariable Cox-models were used to test the independent prognostic significance of each variable. Reported P values are two-sided.

### Results

### GEICAM 2006-03 dataset

Sixty-three pre- and post-menopausal patients were evaluated in this study (**Table 1**). Most patients presented ductal carcinomas (83%), tumor sizes of 2-5 cm (76%), histological grade 3 tumors (59%), clinical node-negative disease (54%) and luminal disease by PAM50 (84%). Following chemotherapy, Luminal B tumors showed higher Miller-Payne response than Luminal A disease (mean 2.0 vs. 1.4, P=0.048). However, no difference in response between the two luminal subtypes was observed following endocrine therapy (P=0.407). In addition, no statistical significant interaction (P=0.429) between subtype and treatment (endocrine vs. chemotherapy) for tumor response was observed. Interestingly, the only patient that achieved a pCR (i.e. Miller-Payne Grade 5) had a Basal-like tumor and was contained within the chemotherapy arm.

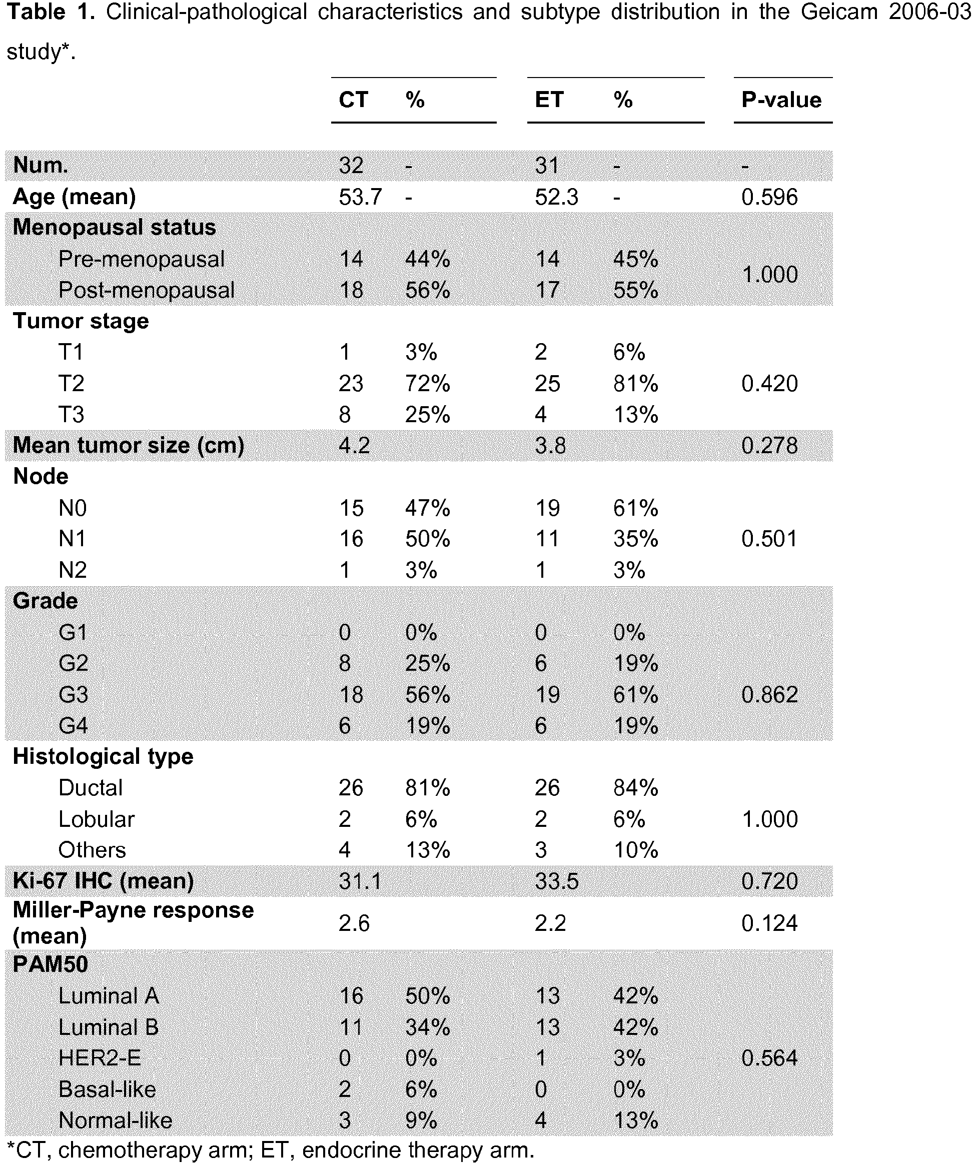

### Gene expression association with treatment sensitivity

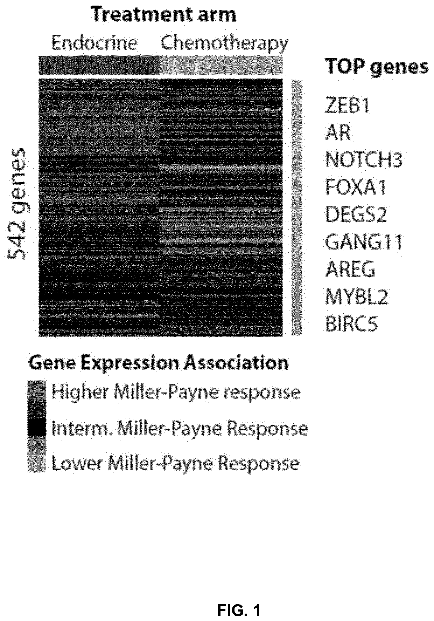

To understand the biology associated with either chemotherapy or endocrine sensitivity within HR+/HER2- disease, we explored the association between the expression of 543 breast cancer-related genes and Miller-Payne response in each treatment arm. High expression of 70 (12.9%) and 17 (3.1%) genes was found significantly associated (P<0.05 uncorrected for multiple comparisons) with response after endocrine therapy and chemotherapy, respectively. The gene list associated with endocrine therapy response was enriched for the following biological processes vasculature development (e.g. AKT1 and catenin beta 1), tube development (e.g. FOXA1 and gremlin 1) and cell growth (e.g. androgen receptor and fibroblast growth factor receptor 1). On the other hand, the gene list associated with chemotherapy response was enriched for cell cycle (e.g. EXO1 and MKI67) and extracellular matrix (e.g. netrin 4 and thrombospondin 1). We then evaluated the interaction between the expressions of each individual gene with response to therapy (endocrine vs. chemotherapy). Interestingly, 41 of the 70 genes associated with response to endocrine therapy, and 8 of 17 genes associated with chemotherapy response, showed a significant interaction with treatment (P<0.05 uncorrected for multiple comparisons). Thus, the biological factors associated with endocrine sensitivity seemed to be associated, at the same time, with chemotherapy resistance, and vice versa. Indeed, an overall inverse pattern was observed between expression of most genes and response to treatment **(****Fig. 1****).**

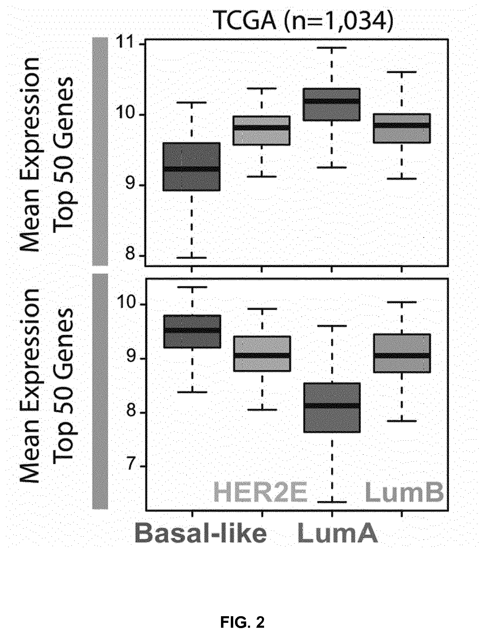

To further understand the biological factors associated with treatment response, we evaluated the mean expression of genes associated with high endocrine but low chemotherapy sensitivity, or low endocrine but high chemotherapy sensitivity, across 1,034 primary tumors representing all intrinsic molecular subtypes of breast cancer **(****Fig. 2****).** The results revealed that the biology associated with chemo-endocrine sensitivity is mostly driven by the Luminal A (i.e. high endocrine but low chemotherapy sensitive) vs. Basal-like biology (i.e. low endocrine but high chemotherapy sensitive).

### Development of a PAM50-based CES

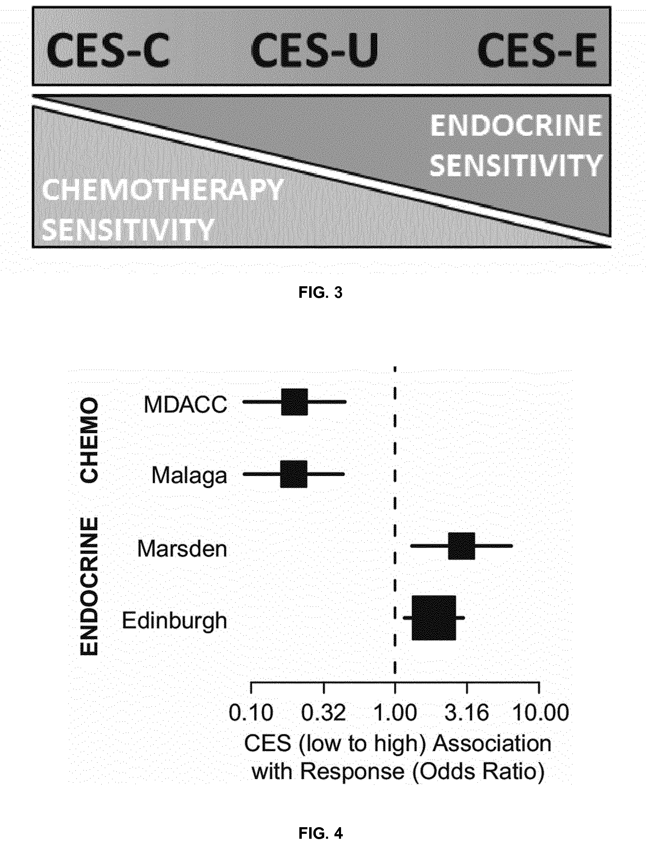

Capturing the relative differences in the Luminal A vs. Basal-like biology within HR+/HER2-could help better predict endocrine and chemotherapy sensitivity. To capture this biological state in each tumor, we obtained, from the PAM50 classification algorithm, the correlation coefficients (CC) of each sample to the PAM50 Luminal A and Basal-like subtype centroids, and then subtracted the 2 values to create the Chemo-Endocrine Score (CES=CC to Luminal A - CC to Basal-like). Thus, samples with a positive score were identified as being more endocrine sensitive than chemotherapy sensitive, whereas samples with a negative score were identified as being more chemotherapy sensitive (CES-C) than endocrine sensitive (CES-E) **(****Fig. 3****).** From GEICAM 2006-03 samples training, cutoffs based on tertiles groups were determined (CES-E vs. CES uncertain [CES-U] group, cutoff = 0.70; CES-U vs. CES-C group, cutoff =0.30). The interaction of the CES score (as a continuous variable) with treatment in GEICAM 2006-03 trial provides some evidence of association (P=0.059).

### MDACC-based dataset

We evaluated a combined dataset of 272 patients with HR+/HER2- disease treated with anthracycline/taxane-based neoadjuvant chemotherapy across several neoadjuvant trials **(****Fig 4****, Table 2).**

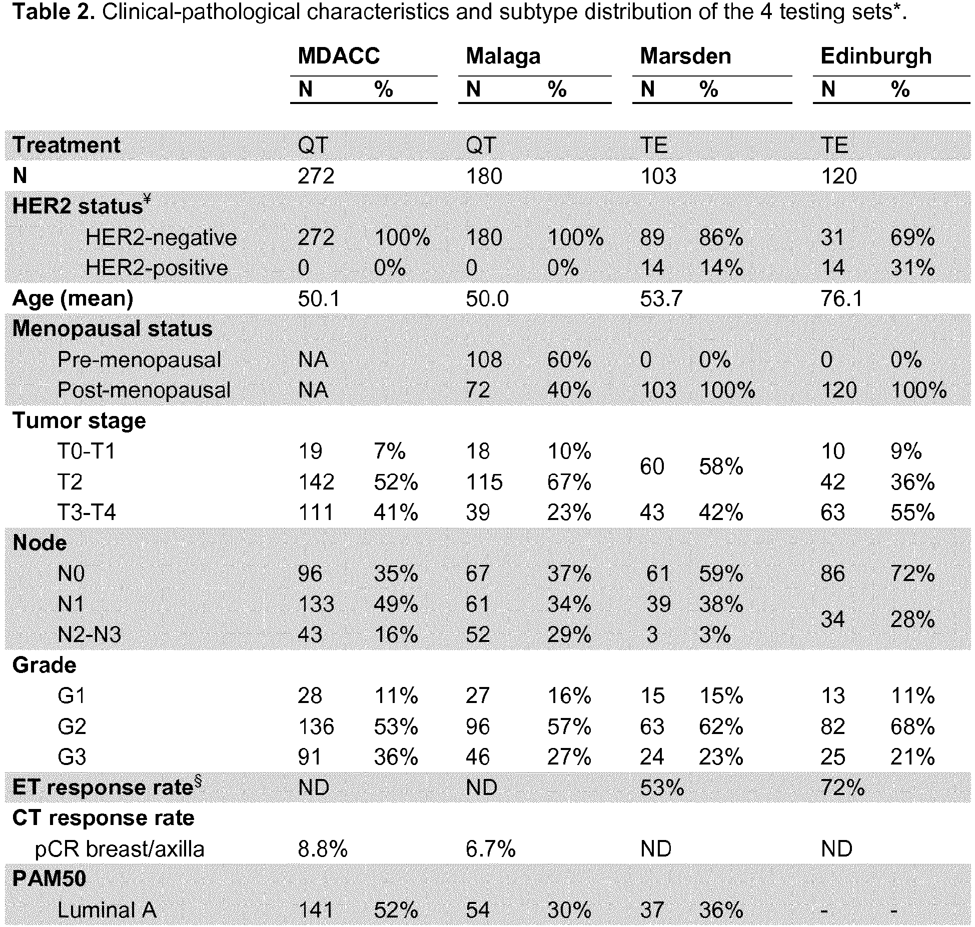

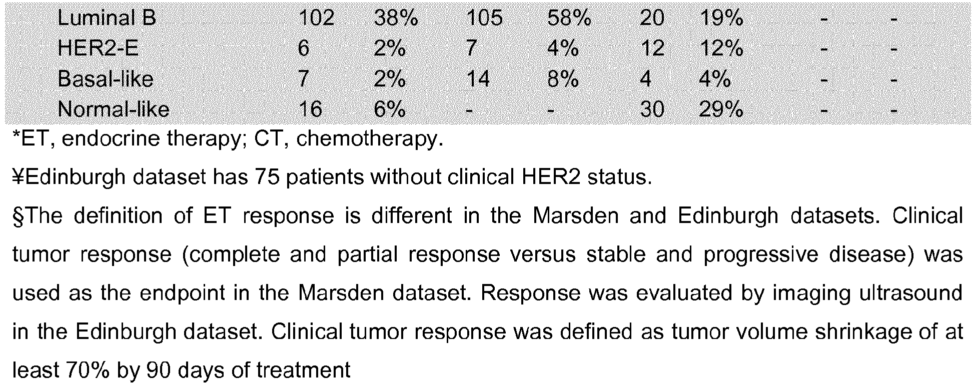

In this dataset, 51.5%, 25.8% and 22.7% of the samples were identified as CES-E, -U and -C, respectively. The rates of pCR across the CES-E, -U and -C groups were 2.4%, 9.0% and 23.7%, respectively (P<0.0001), and were found to be similar even if non-luminal tumors were removed (2.2%, 8.8% and 25.0%). The neoadjuvant chemotherapy predictive ability of CES was independent of clinical pathological variables and intrinsic subtype **(Table 3-4).** Similar results were obtained when residual cancer burden was used as the endpoint **(Tables 5-6).**

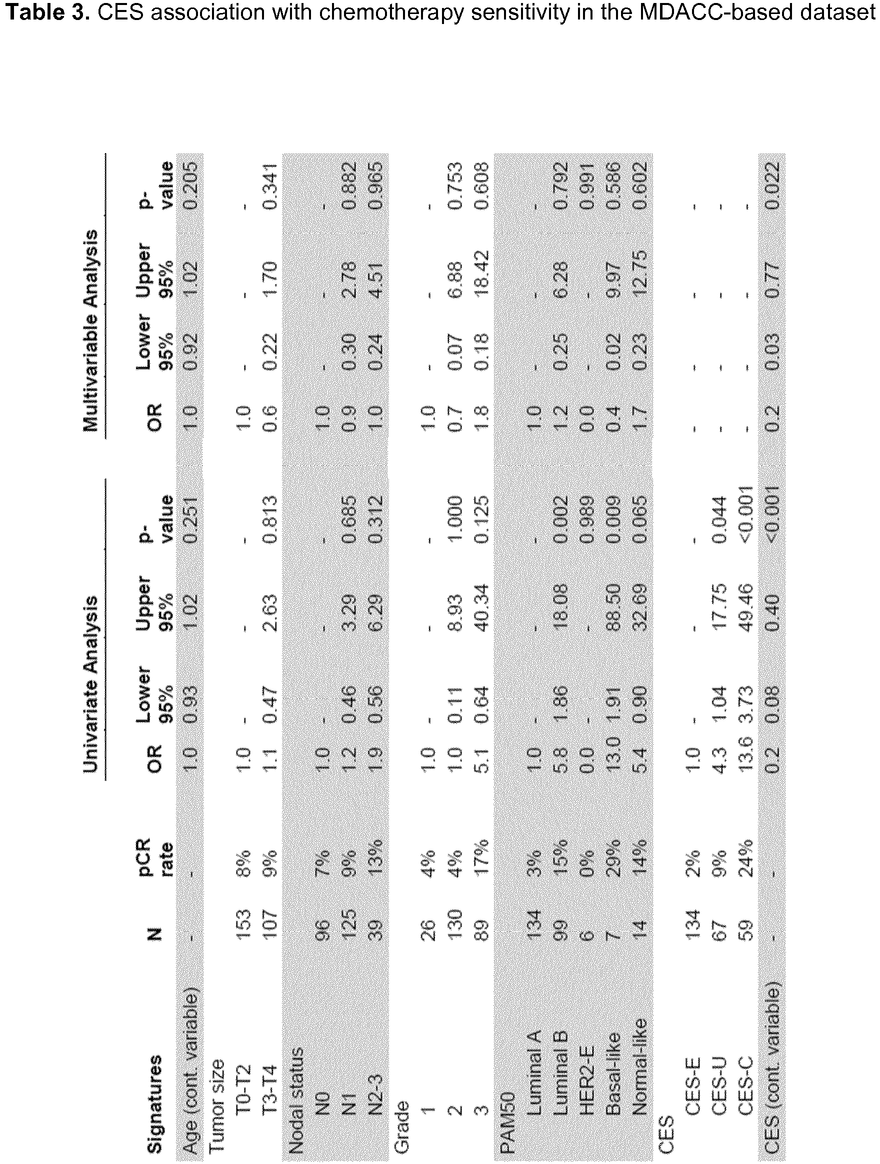

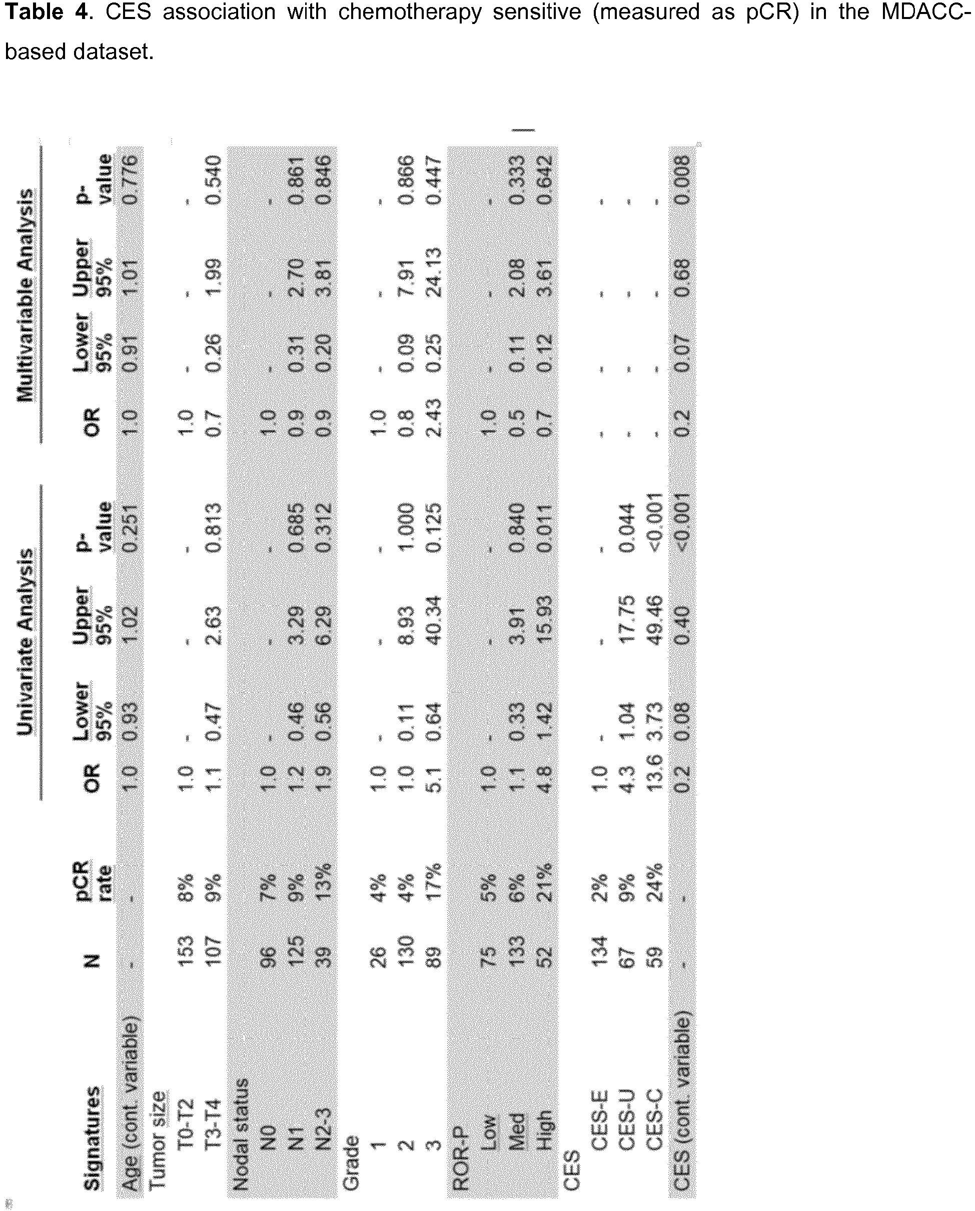

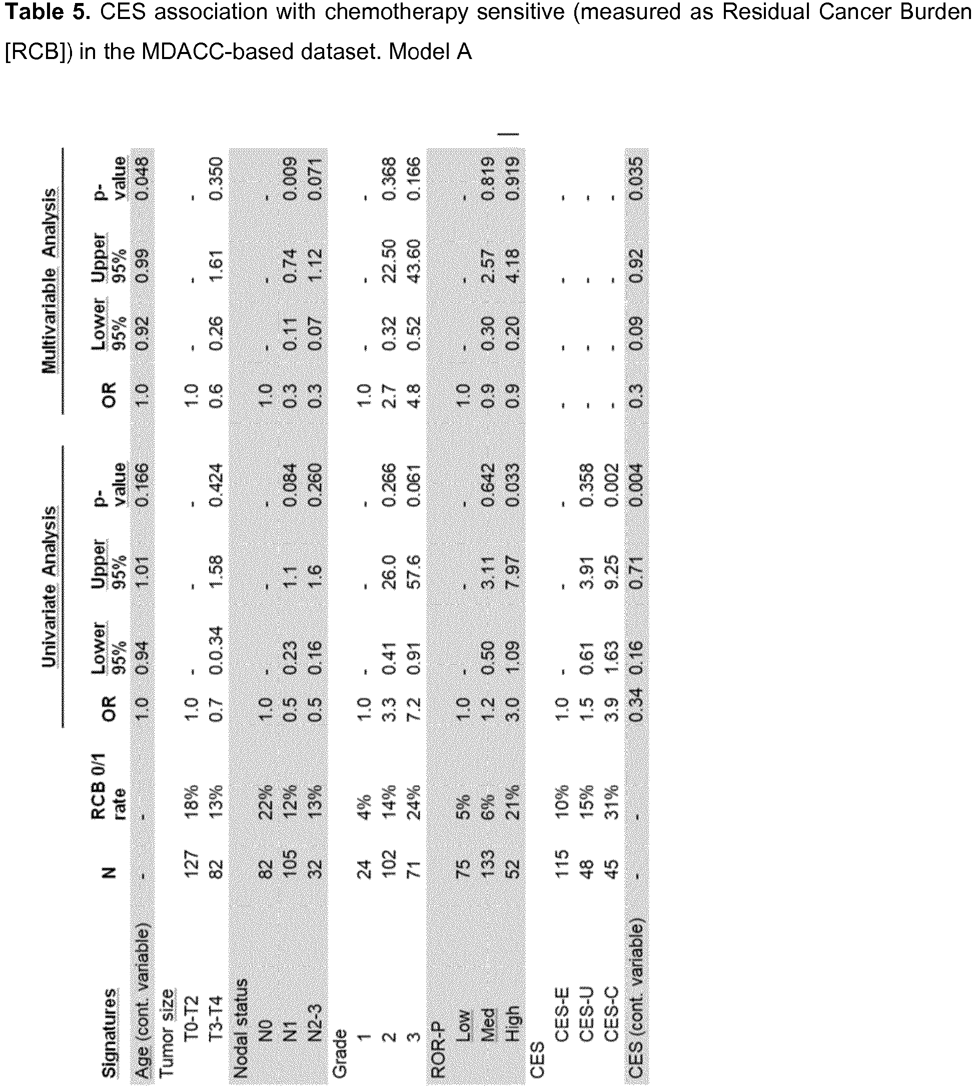

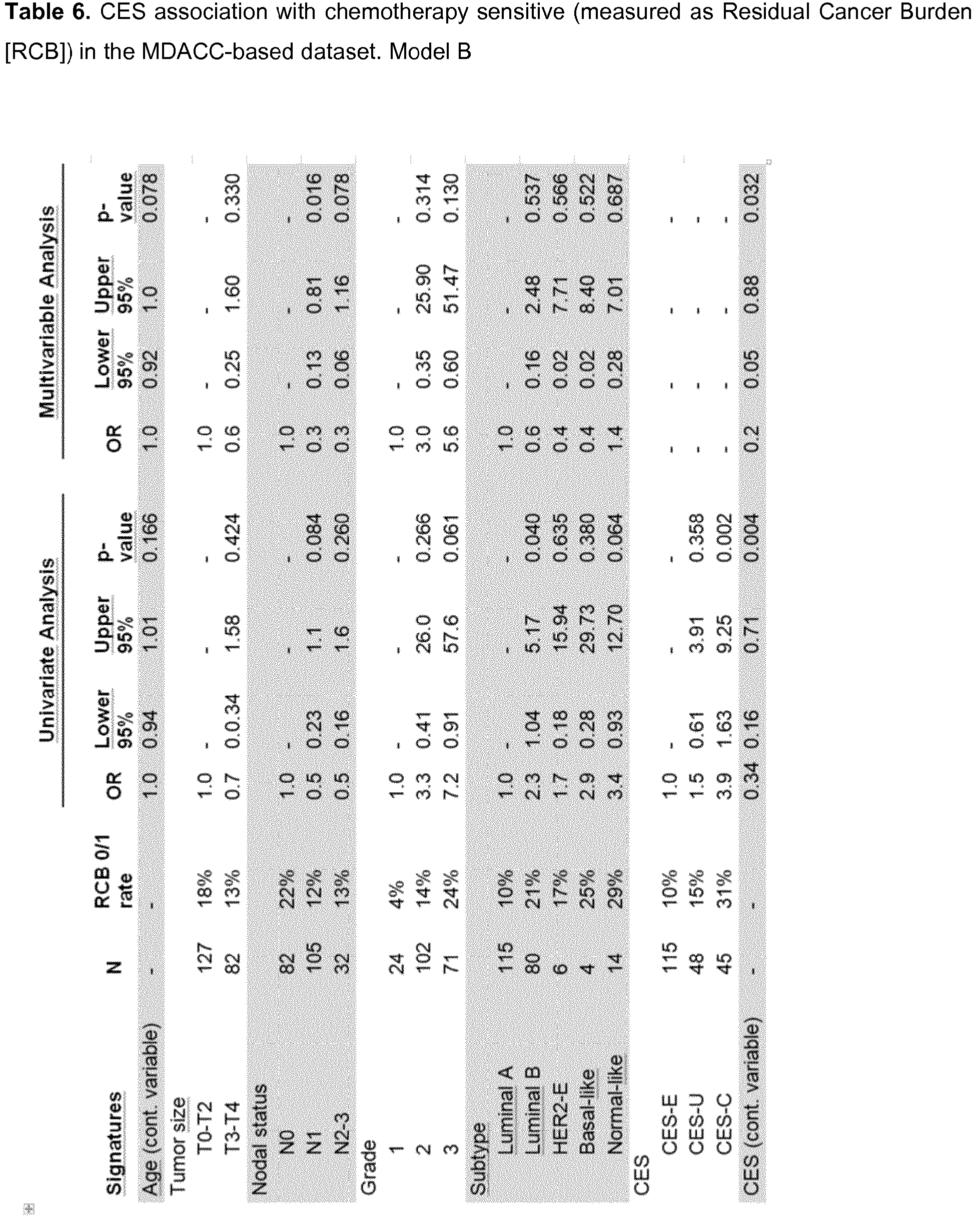

Six gene expression-based signatures (i.e. PAM50 proliferation score, ROR-P, genomic grade index, SET index, chemopredictor, DLDA30 and residual cancer burden [RCB] predictor) have been previously reported in this dataset (Hatzis C, et al. Jama. 2011, 305:1873-1881). In addition, we applied a microarray-based version of OncotypeDX Recurrence Score (Fan C, et al. New England Journal of Medicine. 2006, 355:560-569; Paik S, et al. New England Journal of Medicine. 2004, 351:2817-2826). Here, we evaluated the performance of CES to predict pCR within HR+/HER2- disease compared with these 7 gene signatures. Interestingly, CES provided the highest aROC **(Table 7-15)** either as a continuous variable (aROC=0.770) or as group categories (aROC=0.765). The second most predictive signature was the RCB predictor (aROC=0.740). Of note, RCB predictor was trained using 165 of 272 (60.7%) HR+/HER2-samples from this data set (i.e. the training dataset). When these training samples were removed, CES showed a higher performance either as a continuous variable (aROC=0.805) or as group categories (aROC=0.786) than RCB predictor (aROC=0.640).

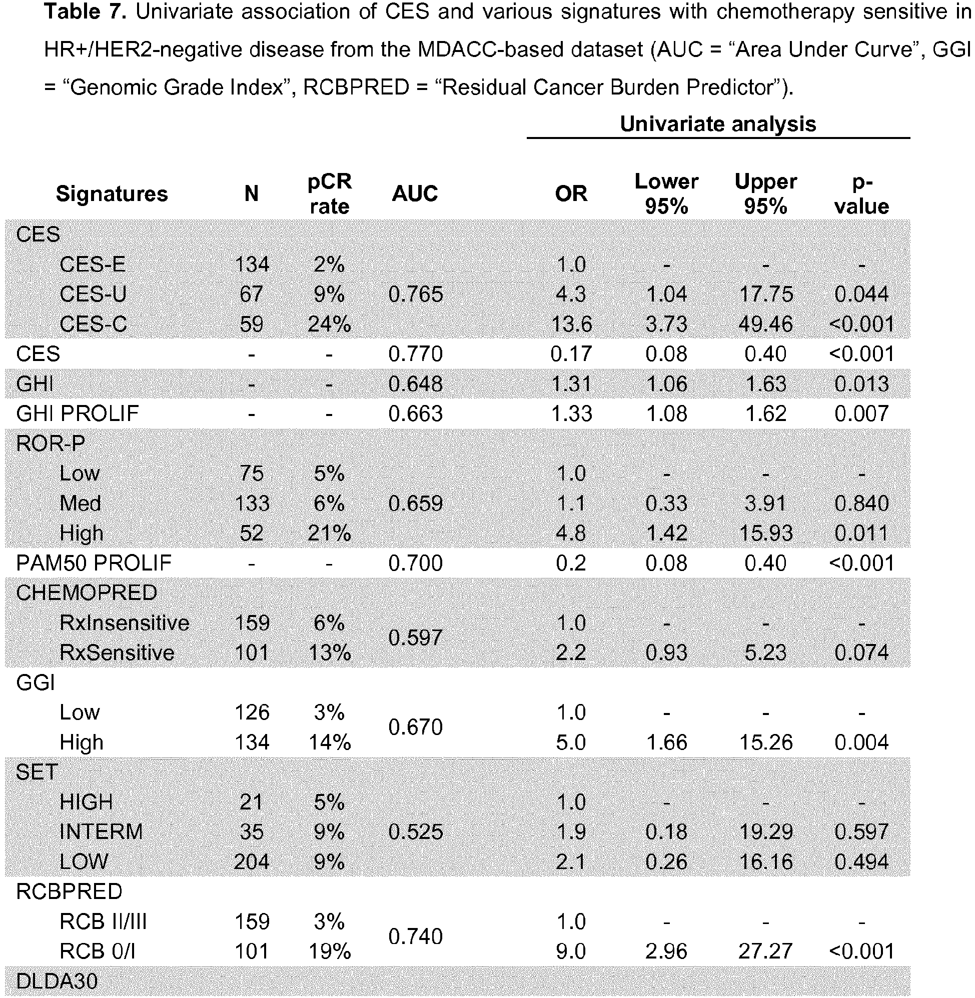

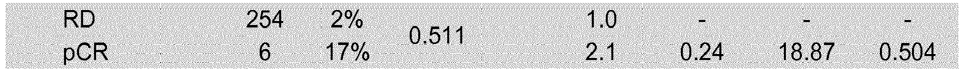

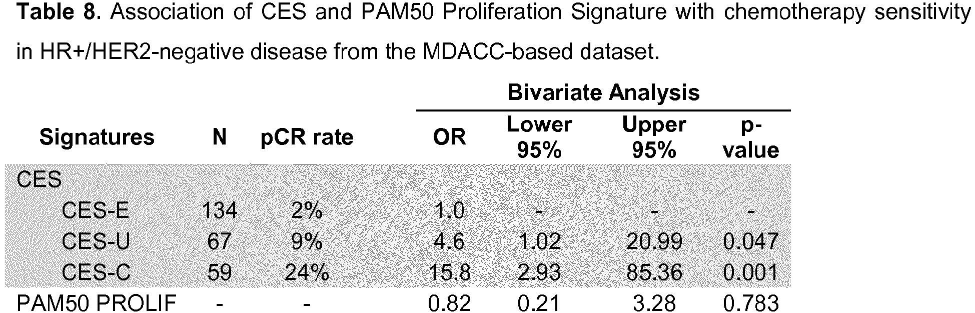

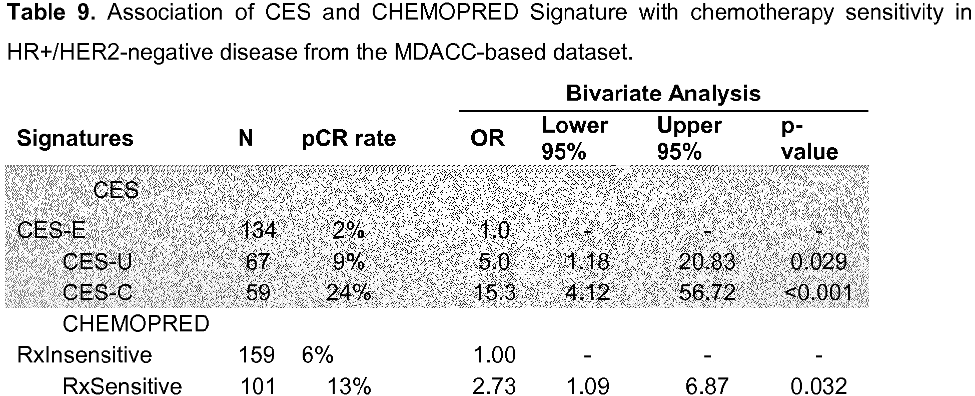

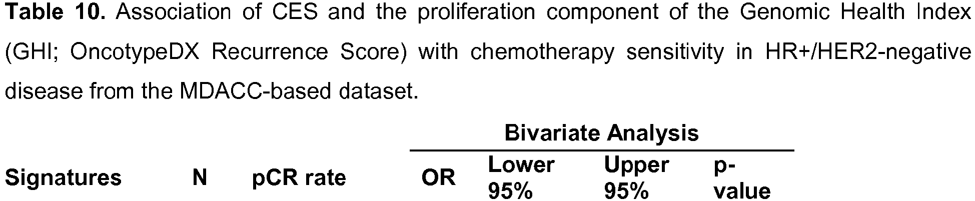

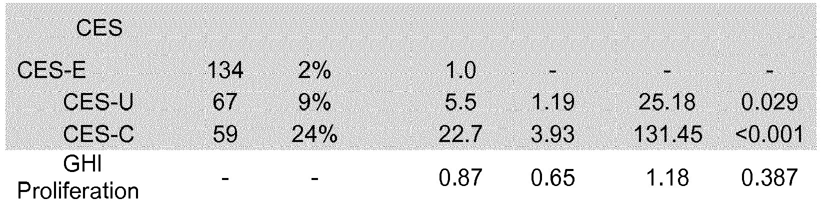

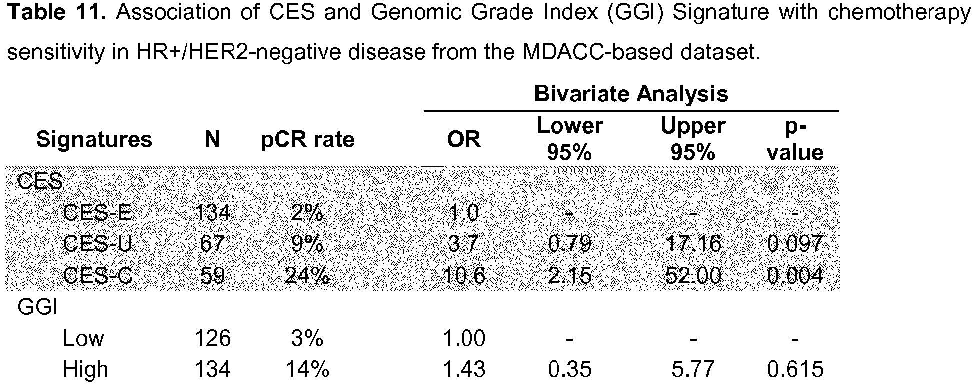

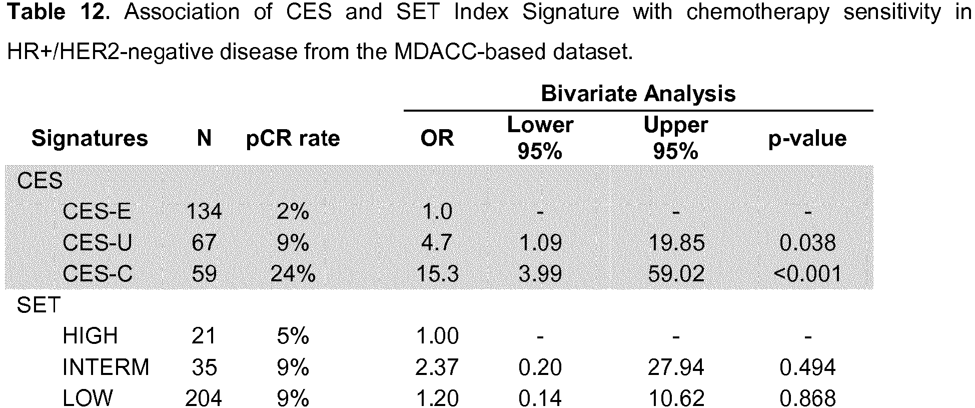

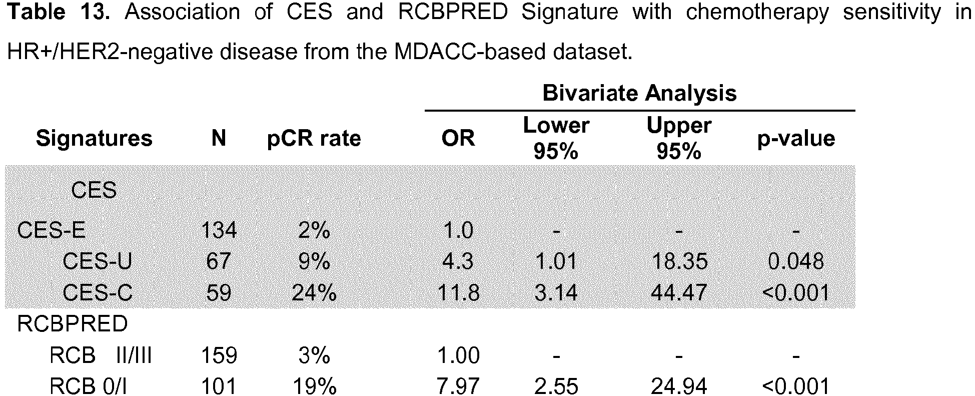

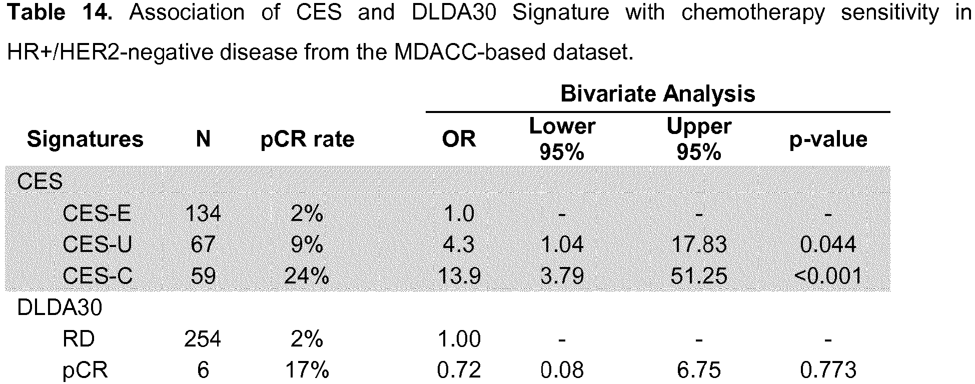

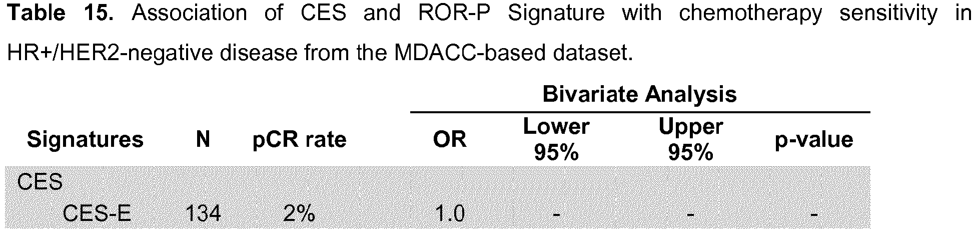

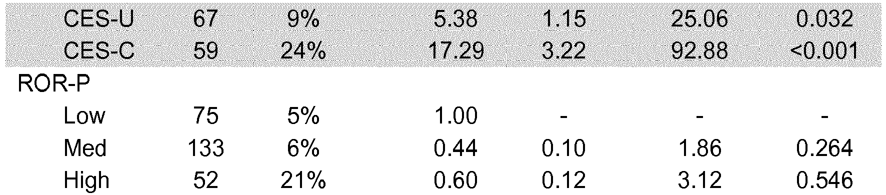

### Malaga-based dataset

We evaluated a dataset of 180 patients with HR+/HER2- disease treated with anthracycline/taxane-based neoadjuvant chemotherapy **(Table 2).** In this dataset, 46.1%, 16.1% and 37.8% of the samples were identified as CES-E, -U and -C, respectively. The pCR and RCB 0/1 rates across the CES-E, -U and -C groups were 2.4%/9.6%, 3.4%/17.2% and 13.2%/30.9%, respectively (P=0.022 and 0.004).

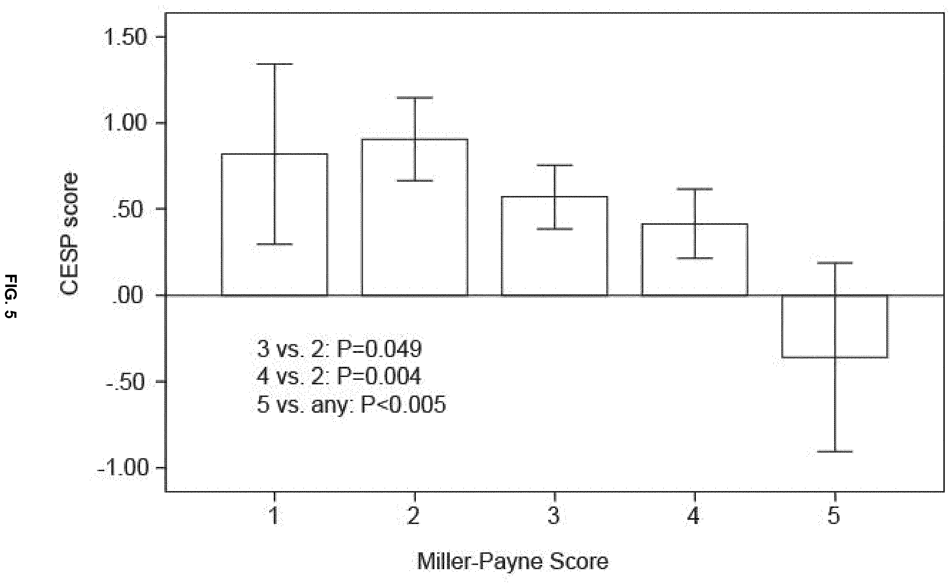

To test the ability of CES to predict chemotherapy response independently of known clinical-pathological variables and intrinsic subtype, we performed a multivariable logistic regression analysis using RCB (0/1 vs. 2/3) as the endpoint since only 12 samples achieved a RCB 0 (i.e. pCR) in this dataset. The results revealed that CES provided independent predictive information beyond intrinsic subtype **(Table 16),** Ki-67 by IHC **(Table 17)** and PAM50 ROR score **(Table 18).** The aROC of CES for predicting RCB 0/1 was 0.746. Finally, we observed a significant association between CES and Miller-Payne response data **(****Fig. 5****).**

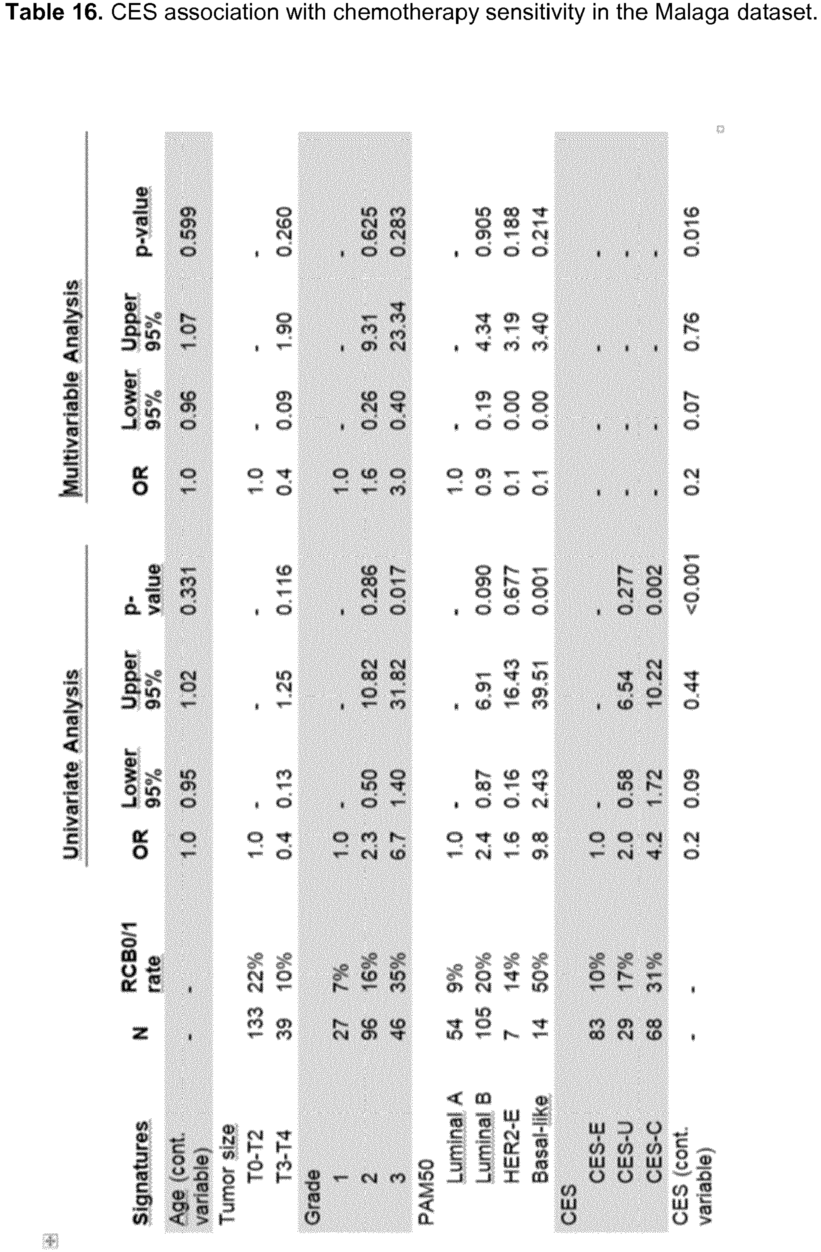

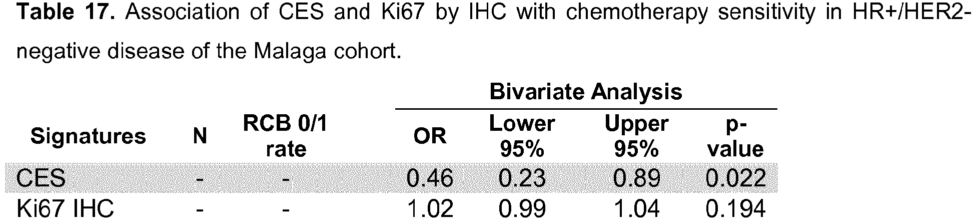

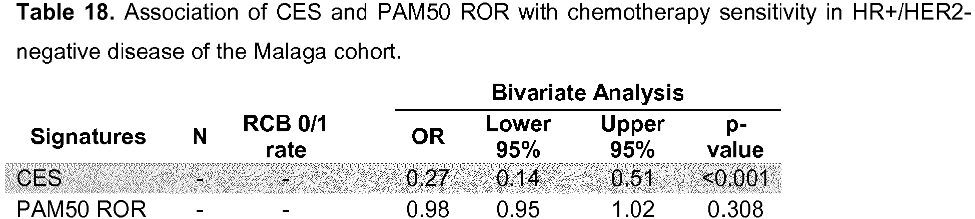

### Marsden-based dataset: CES and endocrine sensitivity

We evaluated a dataset of 103 post-menopausal patients with HR+ disease treated with anastrozole for 16 weeks in the neoadjuvant setting **(Table 2).** In this dataset, 23.5%, 34.3% and 42.2% of samples were identified as CES-E, -U and -C, respectively. Clinical tumor response (complete and partial response versus stable and progressive disease) was used as the endpoint. No pCR was observed in this dataset. The rates of clinical tumor response across the CES-E, -U and -C groups were 75.0%, 48.6% and 44.2%, respectively (P=0.043). CES was found to be the only variable significantly associated with response **(Table 19),** independently of HER2 status **(Tables 19-20).**

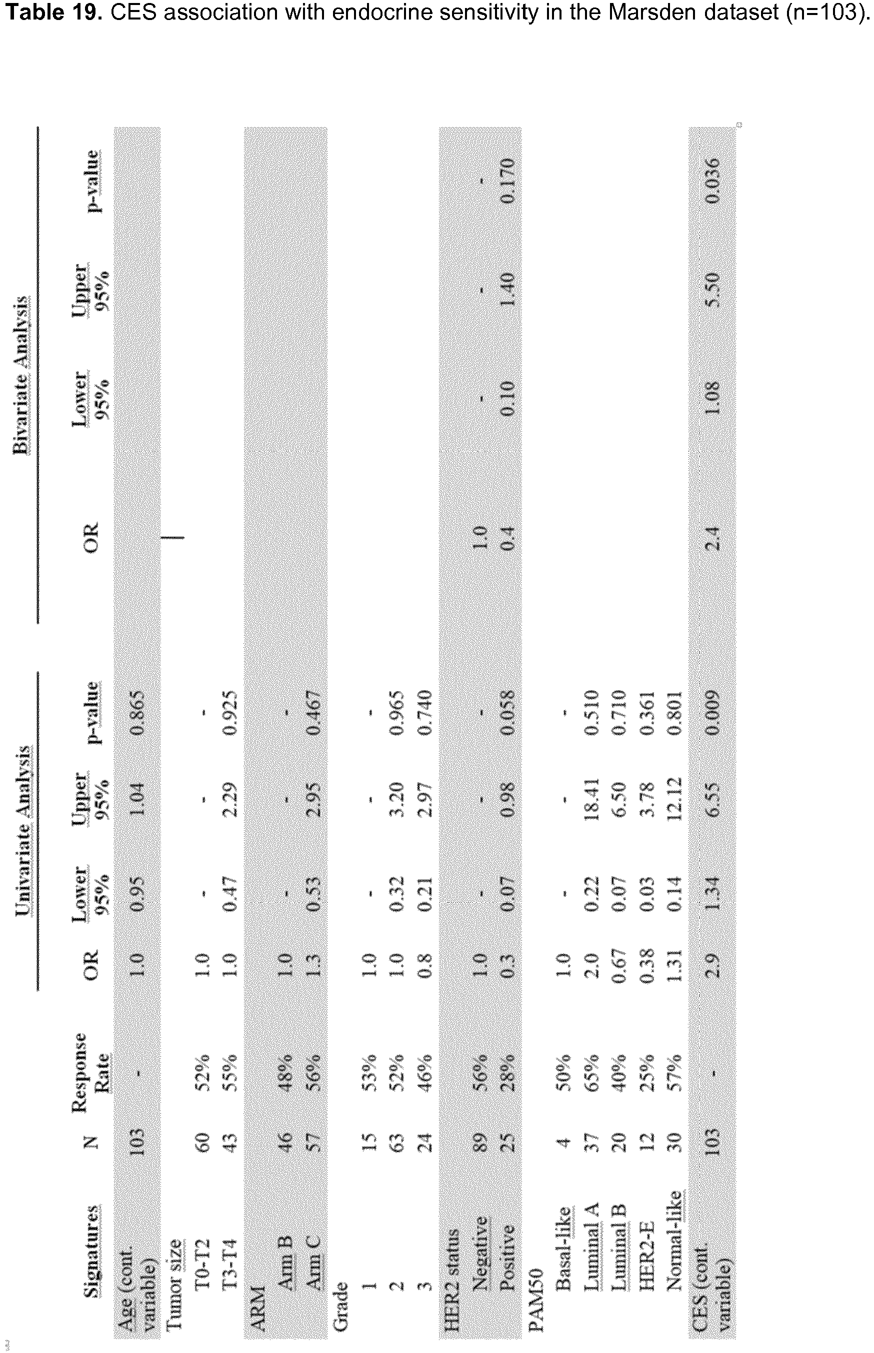

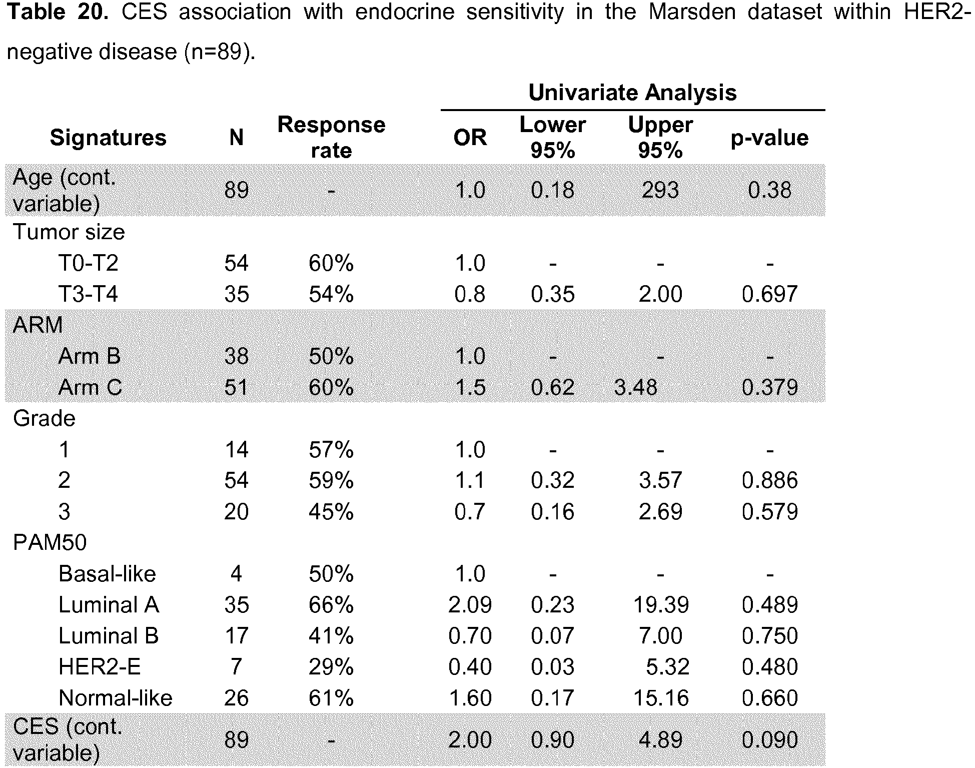

### Edinburgh-based dataset: CES and endocrine sensitivity

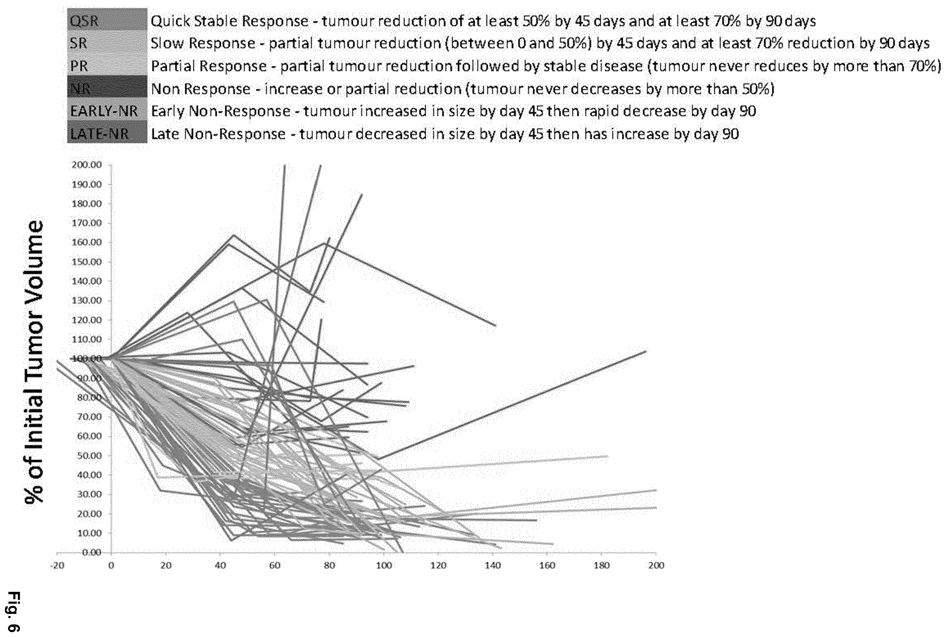

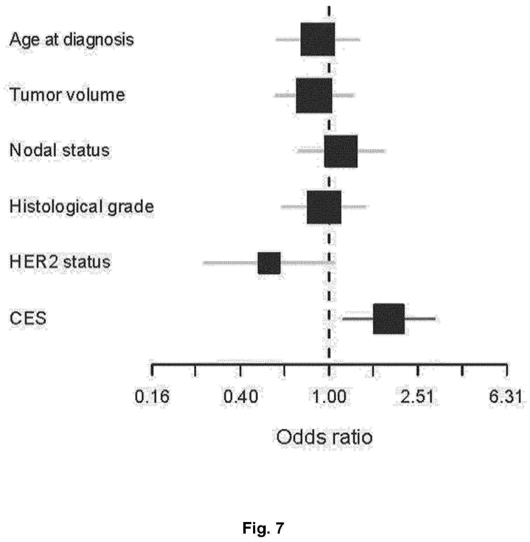

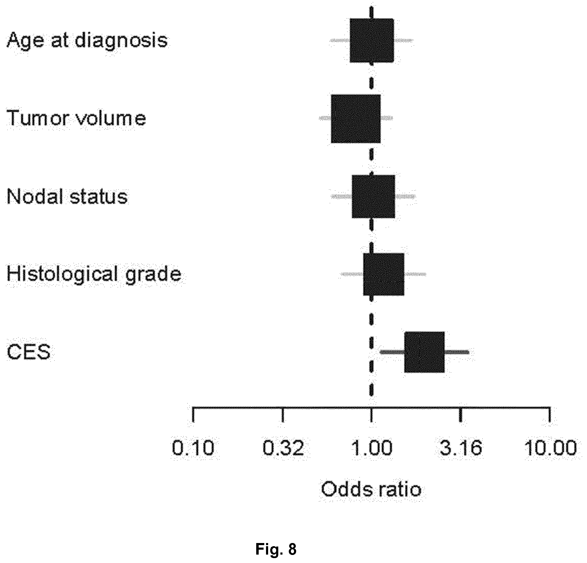

We evaluated a dataset of 120 post-menopausal patients with HR+ disease treated with letrozole for at least 12 weeks in the neoadjuvant setting **(****Fig. 6****).** Two patients of 120 achieved a complete response. Similar to previous results, CES as a continuous variable was found to be the only variable significantly associated with a ≥70% reduction in tumor volume by 90 days **(****Fig. 7****),** even within HER2-negative disease **(****Fig. 8****).**

### Prognosis, intrinsic subtype and chemo-endocrine sensitivity

To better understand the relationship between prognosis, intrinsic biology and chemoendocrine sensitivity, we pulled together PAM50 data from many different datasets for a total of 6,007 primary breast cancers representing all subtypes **(****Fig. 2****).** The results revealed that in the ROR-low group, 94.9% of cases were identified as CES-E and 100% were of the Luminal A subtype. In the ROR-high, 92.1% of the samples were identified as CES-C; non-luminal and Luminal B subtypes represented 64.3% and 35.7% of the RORhigh/CES-C cases, respectively.

In the ROR-intermediate group, high heterogeneity was observed. In terms of intrinsic biology, Luminal A, Luminal B and non-Luminal subtypes represented 44.4%, 31.5% and 24.1%, respectively. In terms of chemo/endocrine-sensitivity, CES-E, CES-U and CES-C represented 40.6%, 30.3% and 29.1%, respectively. As expected, the vast majority of ROR-intermediate/CES-E samples (77.3%) were of the Luminal A subtype.

### Survival outcome of CES within HR+/ROR-intermediate disease

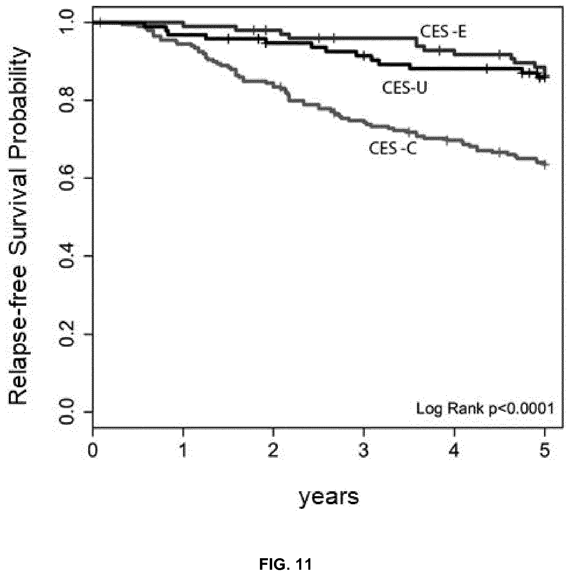

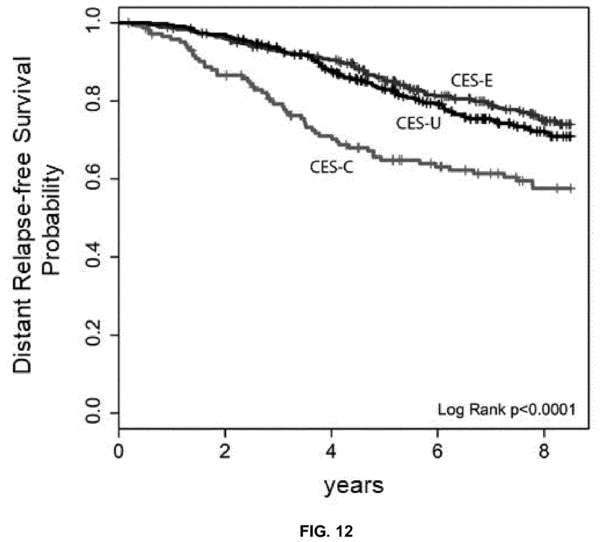

To continue exploring the value of CES within HR+/ROR-intermediate disease, we evaluated the association of CES with survival outcome in HR+/ROR-intermediate early breast cancer in 4 independent datasets of patients treated with no adjuvant systemic therapy (n=189), adjuvant tamoxifen-only (n=846) or adjuvant chemotherapy and endocrine therapy (n=322 and n=148). In patients with node-negative disease treated without adjuvant systemic therapy, CES (as a continuous variable or as group categories) was found significantly associated with distant relapse-free survival **(****Fig. 11****).** The hazard ratio between the CES-C group vs the CES-E group was 2.68 (0.163-0.858 95% confidence interval). Similar results were obtained in the dataset where patients were treated with adjuvant tamoxifen-only **(****Fig. 12****).**

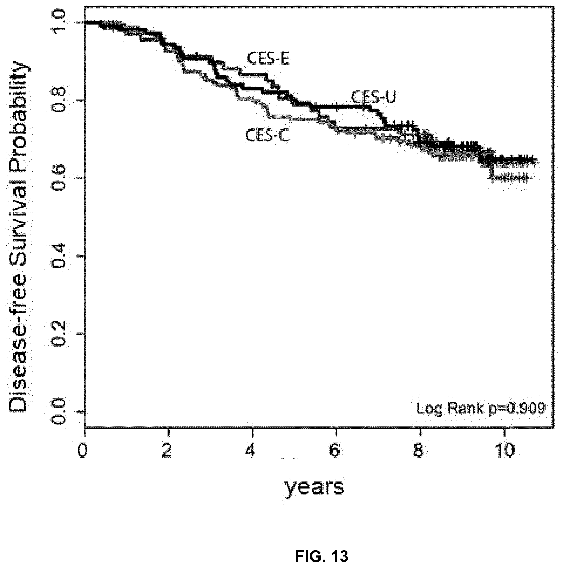

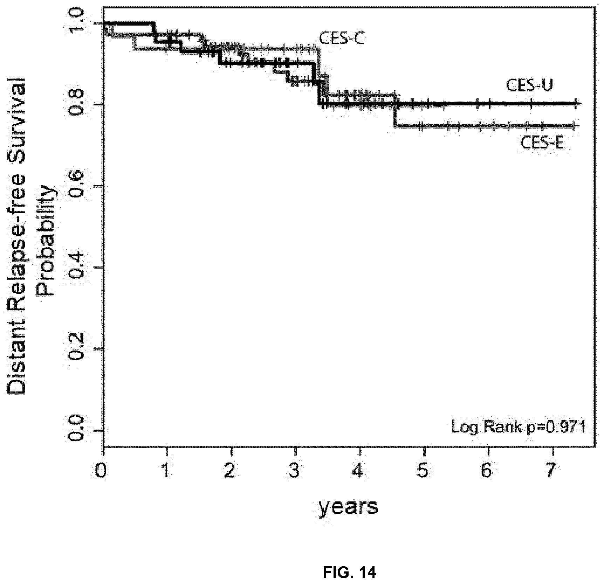

However, CES (as a continuous variable or as group categories) was not found significantly associated with survival outcome in 2 independent cohorts of patients treated with (neo)adjuvant chemotherapy and endocrine therapy (**Fig. 13 - 14****).**

## Claims

1. An *in vitro* method for predicting whether a breast cancer patient is responsive to a chemotherapy or endocrine treatment in an HR+/HER2- isolated sample of the patient classified as ROR intermediate group by the PAM50 kit, wherein said method comprises:
(a) obtaining by the PAM50 kit, the correlation coefficient corresponding to the sample classified as Luminal A intrinsic subtype and the correlation coefficient corresponding to the sample classified as Basal-Like intrinsic subtype, in the isolated sample, and
(b) obtaining the Chemo Endocrine Score (CES) by subtracting the correlation coefficient corresponding to the sample classified as Luminal A subtype from the correlation coefficient corresponding to the sample classified as Basal-Like subtype;
wherein a CES equal or higher than 0.7 is indicative that said patient is responsive to endocrine treatment (CES-E), and wherein a CES equal or lower than 0.3 is indicative that said patient is responsive to chemotherapy treatment (CES-C).

2. An *in vitro* method according to claim 1 wherein the isolated sample is a biopsy sample.

3. The *in vitro* use of the CES for predicting whether a breast cancer patient is responsive to a chemotherapy or endocrine treatment in an HR+/HER2- isolated sample of the patient classified as ROR intermediate group by the PAM50 kit.

4. The *in vitro* use according to claim 3 wherein the CES is obtained by subtracting the correlation coefficient corresponding to the sample classified as Luminal A subtype from the correlation coefficient corresponding to the sample classified as Basal-Like subtype, by the PAM50 kit.

5. The *in vitro* use according to anyone of claims 3 to 4 wherein a CES equal or higher than 0.7 is indicative that said patient is CES-E.

6. The *in vitro* use according to anyone of claims 3 to 4 wherein a CES equal or lower than 0.3 is indicative that said patient is CES-C.

7. The *in vitro* use according to anyone of claims 3 to 6 wherein the isolated sample is a biopsy sample.
